# FASCICULE DE BREVET EUROPEEN

(11) **EP 1 035 846 B1**
(45) Date de publication et mention de la délivrance du brevet: **31.07.2002**
(21) Numéro de dépôt: 98958293.7
(22) Date de dépôt: 01.12.1998
(51) Int. Cl.: A61K 31/20, A61P 29/00

(54) **AGNIM COMME AGENTS ANTI-INFLAMMATOIRES DANS LES TISSUS SUPERFICIELS DES MAMMIFERES**
VERWENDUNG VON NMIFA ALS ENTZÜNDUNGSHEMMER IN OBERFLÄCHLICHEM SAUGETIER-GEWEBE
FATTY ACID UNINTERRUPTED BY A METHYLENE AS ANTI-INFLAMMATORY AGENTS IN SUPERFICIAL TISSUES OF MAMMALS

(30) Priorité: 01.12.1997 EP 97203752
(43) Date de publication de la demande: 20.09.2000
(73) Titulaire: Galderma S.A., 6330 Cham (CH)
(72) Inventeur: BERGER, Alvin, CH-1012 Lausanne (CH); JOMARD, André, F-06460 Saint Vallier de Thiey (FR)
(74) Mandataire: Renard, Emmanuelle
(86) Numéro de dépôt international: FR9802584
(87) Numéro de publication internationale: WO9927924

(56) Documents cités:
- WO-A-95/17897
- FR-A- 2 756 465
- US-A- 5 472 705

## Description

La présente invention concerne la nouvelle utilisation d'un acide gras non interrompu par un méthylène dans la prévention et/ou le traitement de l'inflammation dans les tissus superficiels des mammifères.

### Contexte de l'invention

L'utilisation d'acides gras pour prévenir ou traiter l'inflammation dans les tissus superficiels a été décrite dans la bibliographie.

Par exemple, EP 5 472 705 (Prospa B.V.) décrit de nouvelles compositions pharmaceutiques à utiliser en topique contenant des esters d'acides ω-3-polyinsaturés en forte concentration destinées à traiter le psoriasis, la phlébite et les pathologies apparentées.

EP 582 239 (Rhône-Poulenc Rorer) décrit des compositions pharmaceutiques et cosmétiques topiques contenant de l'acide linoléique ou ses dérivés comme principes actifs, et un véhicule pour transporter le principe actif dans la peau. Ces compositions sont utilisées pour la prophylaxie et le traitement des peaux impures, par exemple les peaux affectées de boutons, de pustules, d'urticaire ou de comédons, de l'acné et des affections cutanées associées à l'acné.

Par ailleurs, EP 5 312 834 (Wooband Land Co.) décrit une composition pharmaceutique pour traiter l'acné contenant de l'acide éicosapentaénoïque et de l'acide α-linolénique, dans un rapport pondéral entre l'acide éicosapentaénoïque et l'acide α-linolénique de 1:0,1 à 20:0,1. Ces acides gras peuvent être extraits de substances naturelles telles que l'huile de poisson et/ou l'huile de perilla, par exemple.

Le terme acide gras non interrompu par un méthylène, dont l'acronyme est AGNIM, désigne un acide gras possédant une série de doubles liaisons dans laquelle au moins une paire adjacente de doubles liaisons est séparée par au moins deux atomes de carbone, à savoir par un groupe autre qu'un seul groupe méthylène. Les AGNIM ont fait l'objet de quelques études seulement dans le but de développer une compréhension de leur incorporation dans les tissus des mammifères et de leur rôle potentiel dans le traitement de certaines maladies. Par exemple, JP 61 058 536 (Nippon Oil) décrit un procédé pour purifier de l'huile de pignon contenant au moins 10% en poids d'acide 5,9,12-cis-octadécatriénoique, qui présente un effet curatif contre l'hypertension artérielle. WO 96 05 164 (Broadbon Nominees Pty) décrit une préparation anti-inflammatoire comprenant une fraction active purifiée, par exemple de l'acide 5,11,14,17-éicosatétraénoïque, isolée d'un extrait lipidique de *Perna canaliculus* ou de *Mytilus edulis*.

WO 95 17 897 (The Regents of the University of California) montre que la grande catégorie des AGNIM, y compris l'acide 5,11,14-éicosatriénoïque, peut être utilisée en quantité efficace pour réprimer les maladies auto-immunes en général, par exemple la polyarthrite rhumatoïde, le lupus érythémateux, la sclérose en plaques, la myasthénie grave et environ 30 autres maladies actuellement connues.

L'incorporation de 5,11,14-éicosatriénoate alimentaire dans différentes classes phospholipidiques et tissus de souris a été étudiée. Les résultats montrent que le fait de les alimenter en 5,11,14-éicosatriénoate conduisait à des taux plus faibles de 20:4n-6 dans le capital de phosphatidylinositol hépatique. Etant donné que les leucotriènes et les prostaglandines ne peuvent pas être formés à partir de 5,11,14-éicosatriénoate en raison de l'absence d'une double liaison interne Δ8 et étant donné que le taux de 20:4n-6 est réduit de façon dramatique dans certains capitaux de phosphatidylinositol, on s'attendait à ce que l'apport alimentaire de 5,11,14-éicosatriénoate puisse altérer le métabolisme des éicosanoïdes, réduisant ainsi l'inflammation potentielle dans le système hépatique (Biochemica et Biophysica Acta, 1085, 371-376, 1991; J. Nutr. Biochem., 4, 409-420, 1993).

Jusqu'à présent, la classe suivante de AGNIM, en particulier l'acide 5,11,14-éicosatriénoïque, n'a pas été signalée comme étant capable d'être incorporée dans la fraction lipidique des tissus superficiels des mammifères. Un effet anti-inflammatoire dans les tissus superficiels des mammifères n'a pas non plus été prévu.

### Résumé de l'invention

On a donc découvert que l'utilisation d'au moins un des AGNIM ayant la formule suivante, dans laquelle le AGNIM est un acide, un sel ou un ester, et R¹ est un groupe alkyle en C₁-C₅ et R² est un groupe alkyle en C₂-C₆, pour la préparation d'une composition destinée à moduler le métabolisme de lipides dans les tissus superficiels des mammifères.

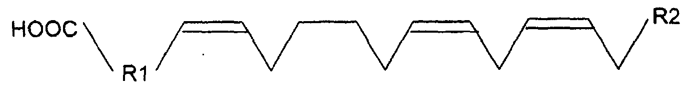

Les AGNIM particulièrement préférés sont ceux dans lesquels R¹ est un groupe alkyle en C₃ et R² est un groupe alkyle en C₂-C₆, ou dans lesquels R² est un groupe alkyle en C₄ et R¹ est un groupe alkyle en C₁-C₅. Celui que l'on préfère le plus est celui dans lequel R¹ est un groupe n-propyle et R² est un groupe n-butyle (l'acide 5,11,14-éicosatnénoïque, appelé également 20:3(5,11,14)).

Les AGNIM peuvent aussi être utilisés dans ce contexte pour préparer une composition destinée à traiter ou prévenir les inflammations dans les tissus superficiels des mammifères en modulant le métabolisme des lipides.
L'invention a aussi pour objet des compositions pharmaceutiques et cosmétiques topiques comprenant les AGNIM de l'invention comme principe actif.
Une composition pharmaceutique, alimentaire ou cosmétique comprend une combinaison d'huile de poisson et des AGNIM.

Les AGNIM offrent des avantages similaires à ceux des huiles de poisson connues de l'homme du métier. Cependant, ils offrent l'avantage d'être moins oxydables que l'huile de poisson, étant donné qu'ils ne possèdent que deux liaisons interrompues par un méthylène, comparées aux 6 liaisons interrompues par un méthylène que comporte l'acide docosahexaénoïque contenu dans l'huile de poisson. De plus, les AGNIM de l'invention ne sont pas un substrat pour la production de prostaglandine et de leucotriène, alors que la prostaglandine et le leucotriène peuvent être formés à partir des acides gras que l'on trouve dans l'huile de poisson, comme le 20:5n-3 et le 22:6n-3. Un autre avantage par rapport à la préparation d'une huile de poisson réside dans l'absence d'odeur "de poisson".

### Description détaillée de l'invention

Par "modulation du métabolisme des lipides", on entend plus particulièrement le catabolisme des médiateurs lipidiques associés à l'inflammation, la différenciation, la prolifération et/ou la fonction de barrière des tissus superficiels.

En outre, l'inflammation de tissus superficiels doit être entendue comme étant le phénomène physiologique faisant appel à la production de cytokines pro-inflammatoires, telles que la cachectine α (TNF-α), par les cellules des tissus superficiels, par exemple les kératinocytes et les cellules épithéliales de la cornée, et les cellules du système immunitaire qui sont contenues dans ces tissus (lymphocytes, cellules de Langerhans et analogues). L'inflammation peut provenir, par exemple, d'une infection, d'une allergie, d'une blessure et d'une exposition à un rayonnement et/ou à des agents irritants et/ou à des agents sensibilisants.

L'acide gras utilisable dans la présente invention est un acide gras polyinsaturé qui est linéaire et monocarboxylique, toutes les doubles liaisons étant des doubles liaisons cis. Plusieurs types de nomenclatures sont utilisés dans cette description, et ce sont les suivantes.
a) La nomenclature pour les composés individuels indiquant le nombre d'atomes de carbone et le nombre et la position des doubles liaisons, un exemple typique étant le "20:4(5,8,11,14)" pour l'acide arachidonique; le nombre précédant les deux points est le nombre total d'atomes de carbone, le nombre situé immédiatement après les deux points est le nombre de doubles liaisons et les nombres entre parenthèses sont les positions des doubles liaisons, en partant de l'extrémité de la chaîne portant le groupe acide carboxylique. Dans tous les composés représentés de cette manière, sauf indication contraire, toutes les doubles liaisons sont cis.
b) La nomenclature pour les classes de composés indiquant la position de la double liaison la plus proche du groupe terminal méthyle, un exemple typique étant "n-3" ou "n-6": le nombre après le tiret désigne la position de la double liaison la plus proche de l'extrémité méthyle de la molécule, en partant de l'extrémité méthyle. Ainsi, l'acide arachidonique se situe dans la classe n-6, de même que l'acide 5,11,14-éicosatriénoïque (20:3(5,11,14)), alors que l'acide 5,8,11,14,17-éicosapentaénoïque (20:5(5,8,11,14,17)) se situe dans la classe n-3. Cette nomenclature est équivalente à la nomenclature "ω" dans la bibliographie, "ω" et "n" étant interchangeables.

Certains des AGNIM de l'invention sont des substances existant dans la nature. D'autres peuvent être synthétisés selon une méthodologie publiée bien connue (voir, par exemple, Evans et al., Chem. Phys. Lipids, 38, 327-342, 1995).

Par exemple, le 20:3(5,11,14) est une substance qui existe dans la nature, généralement sous la forme d'un acide gras dans un mélange d'acides gras. Ce AGNIM se trouve dans toutes sortes de plantes en tant que fraction mineure ou majeure de la composition totale d'acides gras. L'extraction du mélange d'acides gras de leurs sources naturelles et l'extraction du 20:3(5,11,14) des acides gras résultants peuvent toutes deux être réalisées par des procédés classiques d'extraction et de purification bien connus de l'homme du métier.

Les sources naturelles d'acides gras contenant du 20:3(5,11,14) sont principalement des graines végétales et parmi elles tout particulièrement les conifères et les buissons ornementaux. Les huiles de graines de ces plantes sont semblables aux huiles comestibles normales, contenant principalement des acides oléique, linoléique et linolénique, mais contenant aussi des quantités utiles de AGNIM. Le tableau 1 énumère des exemples de graines dont les teneurs en lipides contiennent des quantités significatives de 20:3(5,11,14).

**Tableau 1**

| Source | % de 20:3(5,11,14) parmi les acides gras totaux | Source | % de 20:3(5,11,14) parmi les acides gras totaux |
|---|---|---|---|
| *Juniperis virginiensis* | 14,8 | *Sciadopitys verticallata* | 15 |
| *Plarycladus orientalis* | 3 | *Caltha palustris* | 23 |
| *Juniperis chinesis* | 12,3 | *Calitrus rhomboidea* | 14 |
| *Torreya nucifera* | 7 | *Mortierella alpina** | 7 |
| *Podocarpus nagi* | 24 | *Ephedra campylopoda* | 22 |
| *Anemone rivularis* | 10 | *Anemone leveillei* | 6 |
| *Cimcifuga racemosa* | 6 | *Erantis hyemalis* | 6 |
| *Gingko biloba* | 2,2 | *Pinus silvestris* | 7 |

| | | | |
|---|---|---|---|
| * voir le brevet japonais JP 5276964 (Suntory Ldt.) | | | |

On peut réaliser la purification du 20:3(5,11,14) en particulier (1) en choisissant une source de graines de départ à haute teneur en graisse totale et à haute teneur en 20:3(5,11,14), mais ne contenant pas d'autres triènes contaminants, en particulier de l'acide α-linolénique (18:3n-3) et de l'acide γ-linolénique (18:3n-6) (*Podocarpus nagi*, tableau 1, en est un exemple); (2) en extrayant les lipides avec de l'isopropanol et du chloroforme selon le procédé de Nichols (Biochim. Biophys. Acta 70: 417, 1963), (3) par des procédures classiques de décreusage et de décoloration; (4) en préparant des esters méthyliques avec 2% d'acide sulfurique méthanolique, selon le procédé de Christie (p. 52-53, dans Lipid Analysis, Pergamon Press, Oxford, 1982); (5) en éluant l'ester méthylique de 20:3(5,11,14) sur une colonne de Florisil lavée à l'acide imprégnée de nitrate d'argent avec un mélange hexane/éther de 9:1 à 8:2 (volume/volume) (d'après Carroll, J. Am. Oil Chem. Soc. 40: 413, 1963; Wilner, Chem. Ind. (Lond) octobre, 30: 1839, 1965; Merck ChromNews 4(I), 1995; Anderson, J. Lipid Res. 6: 577, 1965; et Teshima, Bull. Jap. Soc. Scien. Fish. 44: 927, 1978); (6) en éliminant les ions argent contaminants par le procédé de Äkesson (Eur. J. Biochem. 9: 463, 1969); et (7) éventuellement en convertissant à nouveau l'ester méthylique pour avoir la forme acide libre par saponification dans de l'hydroxyde de potassium 1M dans de l'éthanol à 95% d'après Christie (p. 51-52, dans Lipid Analysis, Pergamon Press, Oxford, 1982).

Dans le contexte de la présente invention, les AGNIM peuvent être utilisés sous forme acide, sel ou ester, par exemple sous forme d'ester méthylique, d'ester éthylique, de mono-, di- ou triglycéride ou d'ester phospholipidique. Les AGNIM de l'invention peuvent être utilisés sous une forme purifiée, ou bien sous forme d'un mélange d'acides gras présent dans une huile extraite d'une des plantes décrites ci-dessus, par exemple.

L'administration des AGNIM peut être faite par des méthodes connues de l'homme du métier, à toutes sortes de tissus superficiels des mammifères, autrement dit aux cellules qui constituent la peau, le cuir chevelu, l'oeil, ou la muqueuse orale, buccale, nasale et vaginale, par exemple.

Les AGNIM peuvent être utilisés pour le traitement ou la prophylaxie de maladies de la peau ou du cuir chevelu, en particulier contre les inflammations associées, par exemple, au psoriasis, à l'érythème (coup de soleil), à l'eczéma, à la dermite séborrhéique, à la pelade, à la mycose, à l'acné et autres dermatoses.

Une composition destinée aux applications en topique peut donc comprendre une quantité efficace des AGNIM en tant que principe actif et un véhicule pour transporter le principe actif dans les tissus superficiels des mammifères.

Le véhicule peut être toute sorte de véhicule connu de l'homme du métier pour des formulations cosmétiques ou pharmaceutiques. Les liposomes peuvent donc être l'un des véhicules utilisés dans ce contexte. Ces molécules englobent un ou plusieurs types de substances diverses, notamment les lipides non polaires, les lipides polaires, les mono- et les diglycérides, les sulfatides, la lysolécithine, les phospholipides, la saponine, les acides biliaires et les sels. Les liposomes peuvent exister sous forme d'émulsions et de mousses, de micelles, de monocouches insolubles, de cristaux liquides, de dispersions de phospholipides et de couches lamellaires. Les AGNIM peuvent être incorporés dans le liposome, éventuellement conjointement avec un ligand ou agent mimétique approprié qui se lie aux récepteurs spécifiques de la cellule.

Les liposomes sont généralement formés de lipides formant des vésicules standard, qui englobent en général les phospholipides portant une charge neutre ou négative, et un stérol tel que le cholestérol. Le choix de lipides est généralement guidé par la prise en compte de divers facteurs tels que, par exemple, la taille des liposomes souhaitée et le besoin de stabilité des liposomes dans la circulation sanguine. Typiquement, le constituant lipidique majeur des liposomes est la phosphatidylcholine. La phosphatidylcholine d'oeuf partiellement hydrogénée en est un exemple typique.

Les liposomes contiennent typiquement environ 5-15 pour cent en moles de phospholipides de charge négative, tels que le phosphatidylglycérol, la phosphatidylsérine ou le phosphatidylinositol. Les phospholipides de charge négative aident à empêcher l'agrégation spontanée des liposomes et donc à diminuer la présence d'agrégats formés à partir de liposomes sous-dimensionnés. Des agents de rigidification des membranes, tels que la sphingomyéline ou un phospholipide neutre saturé, à une concentration d'au moins environ 50 pour cent en moles, et 5-15 pour cent en moles de monosialylganglioside, peuvent aussi être incorporés pour procurer une plus grande circulation de la préparation de liposomes dans la circulation sanguine.

Les suspensions de liposomes peuvent aussi contenir des agents protecteurs de lipides pour protéger les lipides de dégâts radicalaires et peroxydants contre les lipides durant le stockage. Des exemples de ces agents sont les extincteurs de radicaux libres lipophiles tels que l'α-tocophérol et les chélateurs spécifiques du fer solubles dans l'eau, tels que la ferrioxyamine.

Les liposomes peuvent être préparés par toutes sortes de procédés connus de l'homme du métier. Les liposomes peuvent ensuite être dimensionnés dans une gamme granulométrique souhaitée et avec une distribution granulométrique relativement étroite. Une gamme granulométrique qui permet de stériliser la suspension de liposomes par filtration à travers un filtre classique, par exemple, est d'environ 0,2-0,4 microns (µm).

Les formulations destinées à l'administration comprennent en général une solution du composé en solution ou en suspension dans un véhicule acceptable, dont les choix appropriés viendront facilement à l'esprit de l'homme du métier. Les formulations peuvent par ailleurs contenir des substances auxiliaires pharmaceutiquement acceptables éventuellement nécessaires pour approcher les conditions physiologiques, telles que des agents d'ajustement du pH et des agents tampons, des agents d'ajustement de la tonicité et des agents mouillants. Les formulations peuvent également avantageusement comprendre des antioxydants, de préférence des anti-oxydants liposolubles, tels que notamment le tocophérol, l'acétate de tocophérol, le butyl-hydroxy toluène, le butyl hydroxy anisole, le palmitate d'ascorbyle et leurs mélanges.

La composition cosmétique ou pharmaceutique peut être appliquée directement sur les tissus superficiels de telle sorte que les AGNIM diffusent à travers les cellules et y soient administrés. Cette composition peut aussi être injectée sous les tissus superficiels, par exemple au moyen d'une injection sous-cutanée. Dans les deux cas, l'application est considérée comme topique, autrement dit est appliquée directement sur ou sous les tissus superficiels.

L'application des AGNIM peut aussi être étendue aux inflammations de l'oeil, plus particulièrement de la cornée, ainsi que des muqueuses, plus particulièrement des muqueuses orale, nasale, buccale, anale et vaginale. Les compositions administrées oralement peuvent affecter directement les muqueuses buccale, oesophagique, gastrique et intestinale, et/ou peuvent passer dans la circulation sanguine et être transportées directement, par exemple, aux cellules de la peau, de l'oeil ou de la muqueuse.

Cette composition peut aussi être appliquée dans les passages nasaux au moyen d'un diffuseur, d'un gel et/ou d'un liquide physiologique servant de façon classique à laver les passages nasaux.

Il convient d'insister sur le fait, que, en fonction des formes galéniques habituellement utilisées pour une application en topique ou orale, autrement dit suivant qu'il s'agit d'une forme alimentaire, cosmétique et/ou pharmaceutique, la quantité des AGNIM nécessaire et suffisante pour observer un effet anti-inflammatoire ou un effet de modulateur du métabolisme des lipides peut varier considérablement. L'utilisation des AGNIM en quantité suffisante pour le traitement ou la prévention des inflammations et/ou pour les modulations du métabolisme des lipides des tissus superficiels.

Les AGNIM seront donc administrés à l'homme ou à un animal, par exemple sous forme de denrée alimentaire, pour traiter la muqueuse orale. Ils peuvent donc être utilisés pour remplacer les huiles normalement utilisées dans les formulations ou recettes alimentaires ou en tant que partie d'un mélange d'huiles utilisé de cette manière. Cette composition peut être, par exemple, une sauce, telle qu'une sauce à salades, une huile de table, une mayonnaise, une crème glacée, une composition de confiserie ou une pâte à garnir ou à étaler. Le composé peut aussi être administré en tant que partie d'un complément nutritionnel, tel qu'en comprimé ou en gélule, pris par voie orale quotidiennement. Des liants, des matrices et d'autres adjuvants conventionnels que l'on trouve normalement dans les compléments de ces types seront généralement incorporés ici aussi. Les doses typiques de ces procédés peuvent varier largement, mais se situeront le plus souvent dans la gamme d'environ 2 mg par kg de poids corporel à environ 2 000 mg par kg et plus souvent dans la gamme d'environ 5 à environ 500 mg par kg.

La composition alimentaire peut de préférence comprendre un véhicule pour transporter les AGNIM vers le gros intestin ou une autre région de l'appareil gastro-intestinal. Le véhicule peut être un amidon résistant, incorporé dans une proportion de 2 à 20% en poids. L'incorporation peut être réalisée pendant le séchage avec les constituants alimentaires, par lyophilisation par exemple. L'incorporation peut aussi être réalisée par microencapsulation. Un procédé typique de microencapsulation nécessite le préchauffage d'une huile à bas point de fusion au-dessus de son point de fusion (<40°C), puis l'addition des AGNIM au mélange, l'addition également de l'amidon résistant avec les autres ingrédients et agents liants, tels que la gélatine et les gommes, et la pulvérisation de la dispersion dans la tête d'une tour de refroidissement pour permettre à des particules uniformes de se former avec une granulométrie moyenne typiquement dans la gamme de 20-200 microns (µm). Une forme préférée d'amidon résistant est un amidon à forte teneur en amylose, par exemple ceux décrits dans WO 94/03 049 et WO 94/14 342.

Dans le domaine de la cosmétique humaine et animale, l'invention concerne aussi l'utilisation des AGNIM pour la préparation d'une forme galénique habituellement utilisée pour l'application en hygiène topique ou orale, et en particulier sous la forme de solutions huileuses, alcooliques ou aqueuses-alcooliques, de gels, d'émulsions eau dans l'huile ou huile dans l'eau ayant l'aspect d'une crème ou d'un gel, éventuellement capables de mousser, sous la forme d'un aérosol; ou bien également sous la forme de dispersions vésiculaires comprenant des lipides ioniques et/ou non ioniques. Ces formes galéniques sont dans tous les cas préparées par les procédés habituels dans le domaine cosmétique considéré. Cette composition peut être, par exemple, et en particulier, une composition pour nettoyer, protéger, traiter ou soigner le cuir chevelu, pour le visage, pour le cou, pour les mains ou pour le corps (par exemple sous forme de crèmes de type vanishing cream, de crèmes de nuit, de crèmes démaquillantes, d'écrans ou d'huiles solaires, d'huiles pour le corps ou le visage, de laits de toilette, de laits démaquillants ou de laits pour le corps), une composition de maquillage (par exemple un fond de teint), une composition de bronzage artificiel, une composition pour le bain, une composition de shampooing (traitement du cuir chevelu) ou une composition pour l'hygiène buccale (bain de bouche, pâte dentifrice ou chewing-gum).

Dans le domaine de la pharmacie humaine ou animale, en particulier en dermatologie, en oto-rhino-laryngologie, en ophtalmologie, en gastro-entérologie et en gynécologie, cette composition peut être, par exemple, une capsule, une gélule, une émulsion, une pommade, une composition pouvant être injectée sous la peau, un onguent, un sirop, un diffuseur, un lavage oculaire, un shampooing ou un bain de bouche comprenant les AGNIM pour le traitement ou la prophylaxie de la peau, des yeux ou des muqueuses. Les différentes formes galéniques possibles sont dans tous les cas préparées par les procédés habituels du domaine pharmaceutique considéré.

La présente invention est décrite plus en détail par les exemples indiqués ci-dessous. Il va sans dire toutefois que ces exemples ne sont présentés qu'à titre d'illustration du sujet de l'invention dont ils ne constituent aucunement une limitation. Les pourcentages sont donnés en poids, sans indication contraire.

### Exemple 1 Métabolisme du 20:3(5,11,14)

On réalise de la manière suivante la détermination du profil du 5,11,14 dans la lignée des kératinocytes immortalisés humains DK2-NR (décrite dans le brevet PCT/EP 96/05 812).

Des cultures confluentes de DK2-NR sont incubées 4 jours dans du milieu NR-2 (Biofluids Inc., Rockville, MD, USA) ayant une concentration accrue en Ca²⁺ (CaCl₂: 1,5mM). Ensuite, les kératinocytes sont incubés 2 fois 2 jours (changement de milieu avec des acides gras au bout de 2 jours) dans NR-2 contenant 1 mg/ml de sérumalbumine bovine (BSA, acide gras libre, Sigma Inc. St. Louis, USA) et 15mM d'ester méthylique d'acide 5,11,14-éicosatriénoïque (ester 5,11,14) et/ou d'ester méthylique d'acide arachidonique (ester 20:4n-6).

Par ailleurs, les cellules sont préincubées pendant deux jours avec 15mM d'ester 20:4n-6 (voir tableau, n° 6-9) et ensuite 2 x 2 jours avec de l'ester 5,11,14 et avec un mélange d'ester 20:4n-6 et d'ester 5,11,14.

A la fin de la période d'incubation, les cellules sont lavées dans HBSS (solution saline de Hanks équilibrée) (Gibco, Life Technologies) contenant 0,1% de BSA et deux fois dans HBSS. Les cellules sont récoltées par raclage (racle à cellules, Costar Inc.) des cellules dans les boîtes de culture. Les cellules sont centrifugées dans 1 ml de HBSS (1 000 tours/min.). L'extraction cellulaire est réalisée dans un mélange d'hexane et d'isopropanol (2:1 en volume/volume) contenant du 2,6-di-tert-butyl-p-crésol. Les phospholipides sont séparés par chromatographie (gel de silice 60) avec l'éluant chloroforme/acétone (96:4 en volume/volume). La fraction acide gras des phospholipides est ensuite estérifiée par chauffage (100°C) dans une solution contenant 10% de BF₃-méthanol (Supelco, Bellefonte, PA, USA). Les esters méthyliques sont séparés. Les acides gras sont quantifiés avec des étalons internes.

Les résultats énumérés dans le tableau 2 ci-dessous montrent que l'acide 5,11,14-éicosatriénoïque est intégré dans les lipides totaux (3,34%).

**Tableau 2**

| | Traitement avec acides gras | | | Acides gras phospholipidiques dans cellules | | | |
|---|---|---|---|---|---|---|---|
| n° | Jour 0 | Jour 2 | Jour 4 | 5,11,14 % | 20:3n-6 % | 20:4n-6 % | 20:5n-3 % |
| 1 | - | Ethanol | Ethanol | 0,00 | 0,11 | 0,43 | 0,04 |
| 2 | - | Ester 5,11,14 | Ester 5,11,14 | 3,34 | 0,17 | 0,47 | 0,06 |
| 3 | - | Ester 20:4n-6 | Ester 20:4n-6 | 0,30 | 0,28 | 8,25 | 0,04 |
| 4 | - | Ester 5,11,14 ester 20:4n-6 | Ester 5,11,14 ester 20:4n-6 | 1,04 | 0,58 | 9,56 | 0,00 |
| 5 | - | Ester 20:4n-6 | Ester 20:4n-6 | 0,30 | 0,55 | 10,39 | 0,00 |
| | Préincubation | | | | | | |
| 6 | Ester 20:4n-6 | Ester 5,11,14 | Ester 5,11,14 | 2,12 | 0,38 | 5,30 | 0,03 |
| 7 | Ester 20:4n-6 | Ethanol | Ethanol | 0,00 | 0,30 | 5,57 | 0,01 |
| 8 | Ester 20:4n-6 | Ester 20:4n-6 Ester 5,11,14 | Ester 20:4n-6 Ester 5,11,14 | 0,98 | 1,05 | 12,90 | 0,01 |
| 9 | Ester 20:4n-6 | Ester 20:4n-6 + éthanol | Ester 20:4n-6 + éthanol | 0,09 | 0,85 | 11,80 | 0,03 |

### Exemple 2 Effet de l'acide 5,11,14-éicosatriénoïque sur la sécrétion de TNF-α par les kératinocytes immortalisés humains traités aux ultraviolets B

Des kératinocytes immortalisés humains (DK2-NR) sont incubés dans des boîtes de culture (diamètre 3,5 cm) avec 1,5 ml de milieu NR-2. Après avoir atteint la confluence, la concentration de Ca²⁺ dans le milieu NR-2 est déplacée à 1,5mM. Au bout de 4 jours, les cellules sont incubées 2 x 2 jours dans NR-2 sans hydrocortisone, avec une forte concentration en Ca²⁺ (1,5mM) et divers acides gras (à savoir 18:2n-6, 18:3n-3, 20:3(5,11,14)). A la fin de la période d'incubation, on retire le milieu NR-2 et on le stocke à 37°C pour nourrir à nouveau les cellules après le traitement aux UVB (milieu NR-2 conditionné). Les cultures cellulaires sont lavées 2 fois avec HBSS. Pour le traitement aux UVB, on incube les cellules dans 0,5 ml de HBSS. On traite les cultures cellulaires avec des UVB à 1,5 kJ (Phillips, TL100, émission maximale à 313 nm). Ensuite, on remplace HBSS par le milieu NR-2 conditionné correspondant. On récolte le surnageant au bout de 24 heures et on le stocke à -20°C. La concentration de TNF-α sécrétée dans le surnageant est quantifiée par ELISA (Pelikine Compact, CLB Amsterdam, Pays-Bas).

Comme l'énumère le tableau 3, on peut constater que l'acide 5,11,14-éicosatriénoïque inhibe la sécrétion de TNF-α (9 pg/ml) dans les kératinocytes immortalisés humains traités aux UVB comparés aux témoins (11,6 pg/ml).

**Tableau 3**

| | TNF-α sécrété [pg/ml] | ± Ecart-type |
|---|---|---|
| Pas d'UVB + témoin (EtOH) | 0,0 | 0 |
| UVB + témoin (EtOH) | 11,6 | 0,009 |
| UVB + 5,11,14 | 9,0 | 0,005 |
| UVB + 18:3n-3 (acide α-linolénique) | 14,5 | 0,015 |
| UVB + 18:1n-9 (acide oléique) | 12,1 | 0,005 |
| UVB + hydrocortisone (0,5 mg/ml) | 2,0 | 0,004 |

### Exemple 3 Effet anti-inflammatoire d'autres AGNIM

La détermination de l'effet anti-inflammatoire de 18:3(5,11,14), 18:3(3,9,12), 19:3(5,11,14), 19:3(4,10,13), 21:3(5,11,14), 21:3(6,12,15), 22:3(5,11,14) et de 22:3(7,13,16) a été réalisée dans la lignée de kératinocytes immortalisés humains DK2-NR (décrite dans le brevet PCT/EP 96/05 812), de la manière décrite dans l'exemple 2 ci-dessus. Les résultats montrent la manifestation d'effets anti-inflammatoires.

### Exemple 4 activité anti-inflammatoire de Podocarpus nagi methyl ester après son administration topique chez la souris.

L'activité anti-inflammatoire de *Podocarpus nagi* methyl ester (comportant 26 % de C20 :3 5, 11, 14; et contenant 0.5 % d'α-tocopherol et 0.2 % de palmitate d'ascorbyle) a été évalué dans le test de l'oedème de l'oreille induit par l'acide arachidonique chez la souris.
Le *Podocarpus nagi* methyl ester correspond à l'huile extraite de graines de *Podocarpus nagi,* l'extraction étant réalisée selon la méthode décrite dans les deux paragraphes suivants le tableau 1 de la description.
Le protocole exact est le suivant:
*Podocarpus nagi* methyl ester ou l'huile contrôle de *Podocarpus nagi* est administré topiquement sur l'oreille droite de souris, à la concentration de 20 % (4 microgrammes dans 20 microlitres d'acétone), une fois par jour pendant 5 jours. Chaque groupe comporte 5 souris et un groupe reçoit l'acétone seulement. L'acide arachidonique à la concentration de 2 % dans l'acétone est administré 1 heure après la dernière application des traitements et l'épaisseur de l'oreille est mesurée à l'aide d'un micromètre (oditest) 1 heure après l'administration de l'acide arachidonique.

L'huile contrôle de *Podocarpus nagi* correspond au *Podocarpus nagi* methyl ester tel que décrit ci-dessus, dans lequel le C20 :3 5, 11, 14 a été essentiellement remplacé par l'acide oléïque (C18:1n-9).

### Résultats :

L'oedème de l'oreille correspond à l'augmentation de l'épaisseur de l'oreille par rapport au groupe ayant reçu l'acétone seulement.

| | | | |
|---|---|---|---|
| Produit administré topiquement | OEdème de l'oreille (mm 10⁻²) Moyenne ± esm | Inhibition de l'oedème (%) Moyenne ± esm | test de student (versus groupe acide arachidonique) |
| Acide Arachidonique (AA) 2 % | 11.75 ± 1.75 | - | - |
| Indométhacine 5 % + AA | 1.00 ± 0.48 | 91.49 | P < 0.001 |
| *Podocarpus nagi* methyl ester + AA | 5.00 ± 0.95 | 57.45 | P < 0.01 |
| Huile de *Podocarpus* nagi + AA | 12.00 ± 2.10 | - 2.13 | P > 0.05 (non significatif) |
| esm : erreur standard à la moyenne | | | |

L'indométhacine, anti-inflammatoire non stéroïdien utilisé comme contrôle positif du test à la concentration de 5 % dans l'acétone, inhibe fortement l'oedème induit par l'acide arachidonique. *Podocarpus nagi* methyl ester à la concentration de 20 % inhibe de 57 % l'oedème induit par l'acide arachidonique. Ces résultats montrent que *Podocarpus nagi* methyl ester présente une activité anti-inflammatoire lorqu'il est administré topiquement sur la peau de souris.

### Exemple 5 analyse des lipides dans la peau après administration topique de Podocarpus nagi ethyl ester chez la souris.

Le *Podocarpus nagi* ethyl ester correspond à l'huile extraite de graines de *Podocarpus nagi,* l'extraction étant réalisée selon la méthode décrite dans les deux paragraphes suivants le tableau 1 de la description.

Le protocole exact est le suivant :
*Podocarpus nagi* ethyl ester (avec anti-oxydants) ou la mixture contrôle (de même composition que *Podocarpus nagi* ethyl ester mais dans laquelle le C20 :3 5, 11, 14 est essentiellement remplacé par le C18 : 1n-9) est administré topiquement sur l'oreille droite de souris, à la concentration de 20 % (4 microgrammes dans 20 microlitres d'acétone), une fois par jour pendant 5 jours. Chaque groupe comporte 4 souris. Sur l'oreille gauche, les souris reçoivent de l'acétone seulement.
2 heures après la dernière administration, les souris sont euthanasiées, et on réalise des biopsies des oreilles ainsi traitées pour analyse des lipides cutanés.

L'incorporation du C20 :3 5, 11, 14 dans les phospholipides de la peau de l'oreille des souris à été analysé de la façon suivante :
Extraction des lipides : 20 mg de chaque biopsie est extraite avec 0,8ml H2O, 1ml de CHCI3 et 2ml de MeOH. Après centrifugation (3minutes à 1000g), le résidu est re-extrait avec 1ml de CHCI3, le surnageant est filtré à travers un verre fritté et lavé avec 1ml de KCI à 0,88%. La phase organique est extraite et concentrée sous azote et redissoute dans 2ml de CHCI3

Séparation des phospholipides (PL) et des lipides neutres (NL): Les différentes classes de lipides ont été séparées par chromatographie en phase solide sur des colonnes de 3ml contenant 500mg de silice (Supelco 57010). Les échantillons tels que décrits ci-dessus (dans 2ml de CHCI3) ont été introduits dans ces colonnes lavées. Les NL ont été obtenus avec 2ml de CHCI3 et les PL avec 4ml de CHCl3/MeOH (2/1) et 4 ml de MeOH. Les échantillons sont concentrés sous azote et redissouts dans 10ml de CHCI3.

Chromatographie couche - mince des phospholipides : Les échantillons de PL obtenus ci-dessus sont introduits sur des plaques de chromatographie couche-mince de 10x10 (Merck 1.13727) et les PL sont séparés dans CHCl3/MeOH/acide acétique/H2O (50/37,5/3,5/2,9). Les PL sont identifiés avec des échantillons de référence.

Méthylation des phospholipides : Les échantillons sont ensuite méthylés avec 200 µl de methanolic HCl/hexane (4/1) contenant 0,4 µg de C17:0 pendant 16 heures à 65°C. La réaction est ensuite neutralisée avec 0,5 ml de K2CO3 à 6%. Les methyls esters (ME) sont extraits 2 fois avec 300µl d'hexane, concentrés sous azote et redissouts dans 5µl d'isooctane pour analyse par chromatographie en phase gazeuse.

Chromatographie en phase gazeuse des phospholipides : les ME sont injectés dans l'appareil Hewlett Packard GC Modèle 6890, équipé avec un collecteur d'échantillons et une colonne capillaire.

**RESULTATS :** acides gras dans les pools lipidiques après application topique des ethyl esters de contrôles* (contrôle) et des ethyl esters de *Podocarpus nagi* (Graine) sur l'oreille de souris.

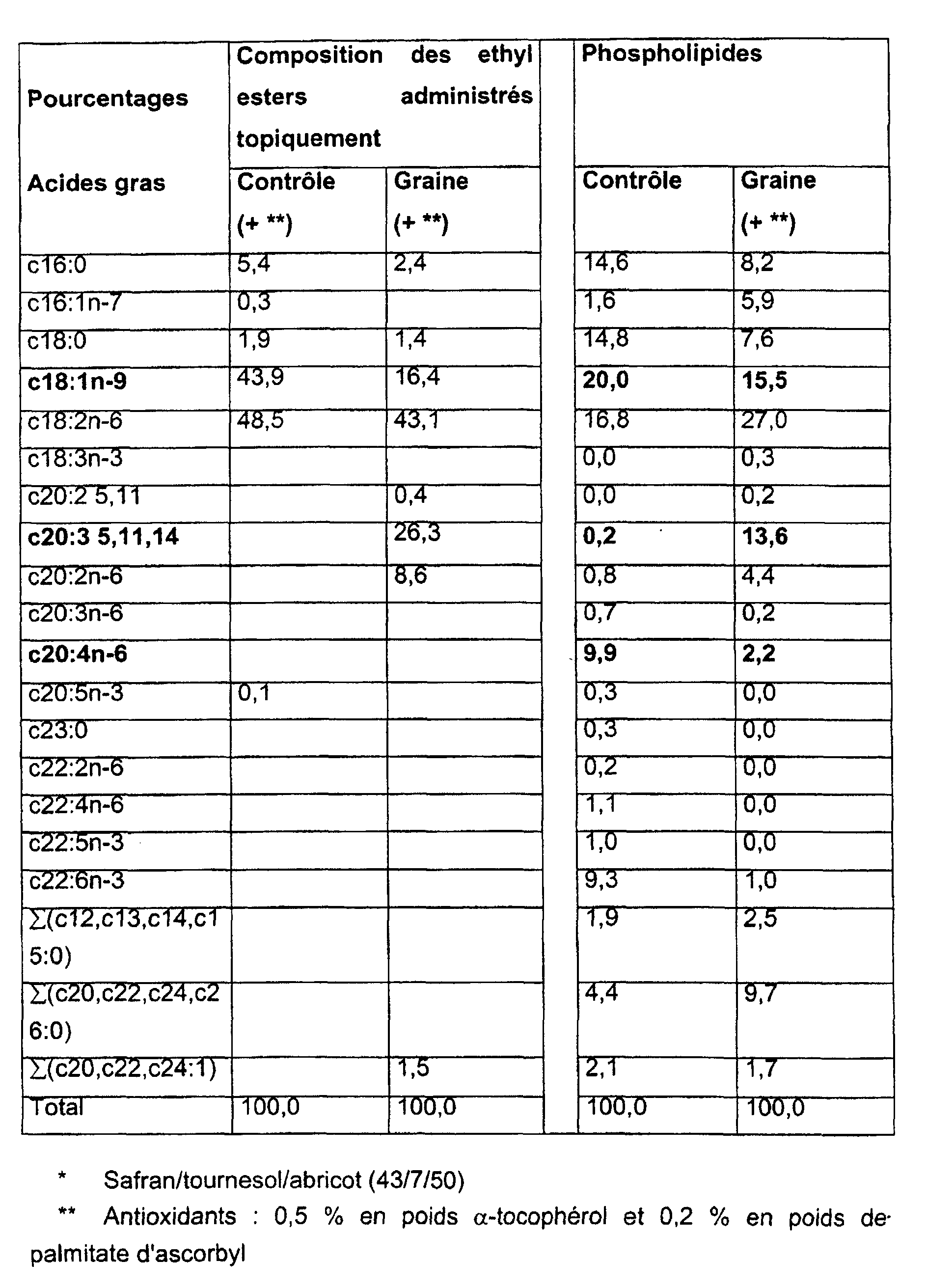

Le C20 :3 5,11,14 augmente dans les phospholipides alors que le 20 :4n-6 (acide arachidonique) diminue. Ces résultats montrent que C20 :3 5, 11, 14 est incorporé dans les phospholipides de la peau après application topique chez la souris.

Le remplacement du 20 :4n-6 par le C20 :3 5, 11, 14 semble corréler avec les propriétés anti-inflammatoires du C20 :3 5, 11, 14 mises en évidence après application topique dans le modèle de l'oedème de l'oreille induit par l'acide arachidonique chez la souris (exemple 4).

### Exemple 6 Lait corporel (émulsion huile dans l'eau)

| Phase huileuse: | |
|---|---|
| Stéarate de glycéryle/stéarate de PEG-100 (Arlacel 165 vendu par ICI) (émulsifiant) | 1% |
| Polysorbate 60 (émulsifiant) | 0,8% |
| Polyisobutène hydrogéné | 2% |
| Acide stéarique | 1% |
| Huile de graines de Ephedra campylopoda | 8% |

| Phase aqueuse: | |
|---|---|
| Glycérine | 3% |
| Carbomère (Carbopol 941, vendu par Goodrich) (épaississant) | 0,3% |
| Triéthanolamine (agent neutralisant) | 0,3% |
| Conservateur | 0,3% |
| Eau | jusqu'à 100% |

On prépare l'émulsion en incorporant la phase huileuse dans la phase aqueuse tout en agitant. Le lait corporel procure une bonne protection de la peau contre les inflammations.

### Exemple 7 Fluide de soin (émulsion huile dans l'eau)

| Phase huileuse: | |
|---|---|
| Sesquistéarate de méthylglucose (émulsifiant) | 2% |
| Cyclométhicone | 13% |
| Huile de graines de Podocarpus nagi | 2% |
| Parfum | 0,2% |
| Sesquistéarate de méthylglucose-PEG 20 (émulsifiant) | 2% |

| Phase aqueuse: | |
|---|---|
| Gomme xanthane (épaississant) | 0,2% |
| Polyacrylamide/isoparaffine C₁₃-C₁₄/laureth-7 (Sepigel 305 vendu par Seppic) (épaississant) | 0,8% |
| Conservateur | 0,3% |
| Eau | jusqu'à 100% |

L'émulsion est préparée de la manière décrite dans l'exemple 6. On obtient un fluide blanc qui procure une bonne protection de la peau contre les inflammations.

### Exemple 8 Crème de soin (émulsion eau dans l'huile)

| Phase huileuse: (A) | |
|---|---|
| Isostéarate de polyglycéryle-4/Cétyl-diméthiconecopolyol/Laurate d'hexyle (Abil WE 09 vendu par Goldschmidt) (émulsifiant) | 4% |
| Isohexadécane | 5% |
| Huile de graines de Caltha palustris | 10% |
| Cyclométhicone | 3,5% |
| Acide n-octanoyl-5-salicylique | 1% |
| Parfum | 0,15% |

| Phase aqueuse: (B) | |
|---|---|
| Glycérine | 10% |
| Gomme de cellulose | 0,5% |
| Sulfate de magnésium | 0,65% |
| Conservateur | 0,3% |
| Eau | jusqu'à 100% |

Pour préparer l'émulsion, on chauffe les constituants de la phase A à 80°C jusqu'à la dissolution complète et on les refroidit à 65°C. On chauffe la phase B à 65°C, on y verse la phase A tout en agitant, puis on réfrigère le mélange. On obtient un fluide blanc qui procure une bonne protection de la peau contre les inflammations.

## Revendications

1. Utilisation d'au moins un des AGNIM ayant la formule suivante, dans laquelle le AGNIM est sous forme d'un acide, d'un sel ou d'un ester, et R¹ est un groupe alkyle en C₁-C₅ et R² est un groupe alkyle en C₂-C₆: 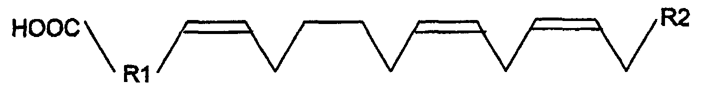 pour la préparation d'une composition destinée à moduler le métabolisme des lipides dans les tissus superficiels des mammifères.

2. Utilisation selon la revendication 1, pour la préparation d'une composition destinée à traiter ou à prévenir les inflammations dans les tissus superficiels des mammifères.

3. Utilisation selon la revendication 1 ou 2, dans laquelle le AGNIM est le 20:3(5,11,14).

## Patentansprüche

1. Verwendung mindestens einer FSNMU der folgenden Formel, wobei die FSNMU in Form einer Säure, eines Salzes oder eines Esters vorliegt, R¹ eine C₁₋₅-Alkylgruppe bedeutet und R² eine C₂₋₆-Alkylgruppe bedeutet: 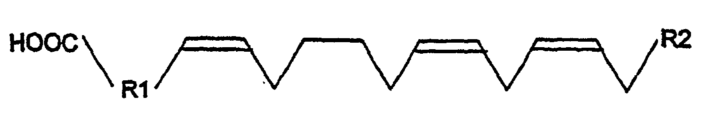 zur Herstellung einer Zusammensetzung, die dafür vorgesehen ist, den Lipidstoffwechsel in den oberflächlichen Geweben von Säugetieren zu modulieren.

2. Verwendung nach Anspruch 1 zur Herstellung einer Zusammensetzung, die zur Behandlung oder Vorbeugung von Entzündungen in den oberflächlichen Geweben von Säugetieren bestimmt ist.

3. Verwendung nach Anspruch 1 oder 2, wobei es sich bei der FSNMU um 20:3(5,11,14) handelt.

## Claims

1. Use of at least one of the NMIFAs having the following formula, wherein the NMIFA is in the form of an acid, a salt or an ester, and R¹ is a C₁-C₅ alkyl group and R² is a C₂-C₆ alkyl group: 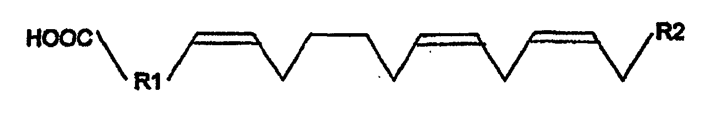 for the preparation of a composition intended to modulate the metabolism of lipids in superficial mammalian tissues.

2. Use according to Claim 1, for the preparation of a composition intended to treat or prevent inflammations in superficial mammalian tissues.

3. Use according to Claim 1 or 2, wherein the NMIFA is 20:3(5,11,14).
